# EUROPÄISCHE PATENTANMELDUNG

(11) **EP 1 764 064 A2**
(43) Veröffentlichungstag der Anmeldung: **21.03.2007**
(21) Anmeldenummer: 06014892.1
(22) Anmeldetag: 18.07.2006
(51) Int. Cl.: A61F 2/38, A61F 2/46

(54) **Vorrichtung und Verfahren zur Bestimmung und Einstellung der optimalen Relativposition einer Funktionsfläche und dementsprechend gestalteten Implantat-Komponenten eines künstlichen Gelenkes**

(30) Priorität: 14.09.2005 DE 102005044044
(71) Anmelder: HJS Gelenk System GmbH, 81925 München (DE)
(72) Erfinder: Nägerl, Hans, 37130 Gleichen (DE)
(74) Vertreter: Scheffler, Jörg

(57) **Zusammenfassung**

Die Erfindung betrifft eine insbesondere zur Anwendung bei einer Endoprothese für das menschliche Kniegelenk bestimmte Vorrichtung (8) und ein Verfahren zur Bestimmung und Einstellung der optimalen Relativposition mehrerer Funktionsflächen eines Gelenkkopfs oder einer Gelenkpfanne des menschlichen oder eines künstlichen Gelenkes. Die Vorrichtung (1) ist mit einem relativ zu einer an einem menschlichen Gelenk oder Knochenbereich fixierbaren Aufnahme (2) mittels einer Schiebeführung (3) beweglichen Kompartiment (4) ausgestattet. Eine sich während der Operation durch die posterior-anterior Bewegung und/oder die medial-laterale Bewegung des Kniegelenkes einstellende Referenzposition des Kompartiments (4) gegenüber der Aufnahme (2) ist dabei mittels einer eine Skalierung (5) aufweisenden Markierung (6) erfassbar.

## Beschreibung

Die Erfindung betrifft eine insbesondere für die Anwendung einer Endoprothese für das menschliche Kniegelenk bestimmte Vorrichtung zur Bestimmung und Einstellung der optimalen Relativposition zumindest einer Funktionsfläche eines künstlichen Gelenkes. Weiterhin betrifft die Erfindung ein Verfahren zur Anwendung bei der Vorrichtung, sowie die Verwendung von Implantaten, die die optimale Gestalt und Relativposition besitzen.

Künstliche Gelenke, insbesondere Kniegelenke, sind bereits Gegenstand vieler Veröffentlichungen. Beispielsweise beschreibt die DE 102 31 538 C1 ein künstliches Gelenk als Endoprothese für das menschliche Kniegelenk, umfassend ein erstes, durch einen ersten Gelenkkopf und eine erste Gelenkpfanne gebildetes, Gelenkkompartiment und ein zweites, durch einen zweiten Gelenkkopf und eine zweite Gelenkpfanne gebildetes, Gelenkkompartiment, wobei dem Gelenkkopf lateral auf der Tibia in der sagittalen Hauptfunktionsebene eine Konvexität sowie in der Transversalen eine Konkavität gegenübersteht und wobei die beiden Kontaktflächen der jeweiligen Gelenkkompartimente in einer Hauptfunktionsebene einen Versatz aufweisen. Hierdurch wird ein für den Patienten besserer Bewegungsablauf unter Annäherung an den natürlichen Bewegungsablauf realisiert.

Die WO 98/20816 betrifft ein künstliches Gelenk bestehend aus mindestens zwei künstlichen Gelenkteilen mit gekrümmten Artikulationsflächen, wobei auf jeder der Artikulationsflächen eine gekrümmte Kontaktlinie ausgebildet ist.

Mit den daraus bekannten künstlichen Gelenken konnte eine optimale Annäherung an den natürlichen Bewegungsablauf erreicht werden.

Als problematisch erweist sich jedoch in der Praxis der Austausch einzelner Kontaktflächen des menschlichen Gelenkes durch Funktionsflächen künstlicher Gelenkelemente, weil für das dadurch erreichbare Bewegungsverhalten die relative Position und Orientierung der künstlichen gegenüber den verbliebenen Funktionsflächen beziehungsweise der künstlichen Funktionsflächen untereinander von entscheidender Bedeutung ist. Diese ist in der Praxis jedoch nicht von dem Geschick des Operateurs, sondern vom Zufall abhängig.

Der Erfindung liegt die Aufgabe zugrunde, eine Vorrichtung zur Bestimmung der optimalen Relativposition mehrerer Funktionsflächen zu schaffen. Weiterhin soll ein Verfahren zur Anwendung bei der Vorrichtung geschaffen werden. Weiterhin dient das Verfahren dazu, optimal eingestellte Implantate zu verwenden.

Die erstgenannte Aufgabe wird erfindungsgemäß mit einer Vorrichtung gemäß den Merkmalen des Anspruchs 1 gelöst. Die weitere Ausgestaltung der Erfindung ist den Unteransprüchen zu entnehmen.

Erfindungsgemäß ist also eine insbesondere zur Anwendung bei einer Endoprothese für das menschliche Kniegelenk bestimmte Vorrichtung zur Bestimmung der optimalen Relativposition mehrerer Funktionsflächen eines Gelenkkopfs und/oder einer Gelenkpfanne des menschlichen und/oder eines künstlichen Gelenkes vorgesehen, wobei die Vorrichtung mit einem relativ zu einer an einem menschlichen Gelenk oder Knochenbereich lösbar fixierbaren Aufnahme beweglichen Kompartiment ausgestattet ist, so dass eine sich durch die Bewegung des Kniegelenkes einstellende Referenzposition des Kompartiments gegenüber der Aufnahme erfassbar ist.

Die Erfindung geht dabei von der Erkenntnis aus, dass die Bestimmung der Relativposition mit hoher Zuverlässigkeit und einem geringem Aufwand dadurch realisiert werden kann, dass vor dem dauerhaften Einsetzen zunächst die Vorrichtung als Einstellhilfe lösbar fixiert wird, deren Kompartiment beweglich ist. Somit führt die nachfolgend eingeleitete Gelenkbewegung zu einer Verlagerung in die optimale Relativposition. Diese Relativposition ist als Referenzposition an der Vorrichtung erkennbar, so dass das künstliche Gelenk aufgrund dieser Referenzposition ausgewählt bzw. angepasst werden kann. Die sich einstellende Relativposition kann wahlweise auch arretierbar sein.

Um diese Aufgabe dem menschlichen Kniegelenk erfüllen zu können ist es vorteilhaft, dass das mediale, also innere Gelenkkompartiment aus Gelenkpfanne und Kopf, und das äußere in sagittaler Richtung (Hauptfunktionsrichtung) konvex-konvex geformt sind, transversal aber der Gelenkkopf konvex und die Tibiafläche konkav gestaltet sind. Diese so gestalteten Flächen lassen sich in einer Viergelenkrelation optimal zu den Bandstrukturen einstellen.

Vorteilhafterweise liegen im Transversalschnitt die Kontaktstrukturen jeweils auf den inneren Abhängen.

Eine vertikale Einstellung ist dabei beispielsweise durch den Einschub von verschieden hohen Inlays oder auch durch einen Schraubmechanismus oder Ähnliches möglich.

Das Kompartiment könnte beispielsweise mittels eines Kugelgelenkes mit mehreren Freiheitsgraden beweglich angeordnet sein. Besonders vorteilhaft ist es jedoch, wenn das Kompartiment in Richtung von zwei Raumachsen beweglich angeordnet ist und dadurch eine einfache Ablesbarkeit der Referenzposition als X- und Y-Koordinaten gestattet.

Hierzu ist es auch besonders vorteilhaft, wenn das Kompartiment mittels einer Schiebeführung mit der Aufnahme verbunden ist, so dass die Relativposition an den beweglichen Bereichen der Schiebeführung direkt ablesbar ist. Dies kann mit einem Rastmechanismus kombiniert werden. Die Schiebeführung kann auch derart ausgeführt sein, dass zur Überwindung eines Reibwiderstandes eine erhöhte Krafteinleitung erforderlich ist, um so eine versehentliche Rückstellbewegung des Kompartimentes auszuschließen. Weiterhin können die Bauelemente unverlierbar angeordnet sein.

Beispielsweise eignet sich zu diesem Zweck eine Abwandlung, bei der das Kompartiment eine insbesondere nutenförmige Ausnehmung aufweist, in welche ein Vorsprung der Aufnahme eingreift, um so den konstruktiven Aufwand zu reduzieren.

Weiterhin erweist es sich als besonders Erfolg versprechend, wenn die Referenzposition durch eine Markierung identifizierbar ist und die Markierung eine Skalierung aufweist, auf deren Basis die Auswahl des geeigneten künstlichen Gelenkes oder einzelner Elemente unmittelbar erfolgen kann.

Eine andere, ebenfalls besonders zweckmäßige Abwandlung der vorliegenden Erfindung wird dadurch erreicht, dass der Markierung ein Sensor zur elektronischen Signalverarbeitung zugeordnet ist, um so die derart erfasste Referenzposition einer elektronischen Datenverarbeitung zuführen zu können und dadurch die Auswahl und Anpassung des künstlichen Gelenkes zu vereinfachen.

Weiterhin ist es besonders günstig, wenn die Vorrichtung einen Sensor zur Erfassung der Relativbewegung des Kompartimentes gegenüber der Aufnahme während der Bewegung des Kniegelenks ausgeführt ist, um so Aufschluss über den Bewegungsablauf und damit die Referenzposition in Abhängigkeit der Winkelstellung des Gelenkes zu erhalten.

Grundsätzlich kann mittels der Vorrichtung die Relativposition einer einzigen künstlichen Funktionsfläche gegenüber verbliebenen Oberflächenbereichen des menschlichen Gelenkes bestimmt werden. Besonders zweckmäßig ist hingegen auch eine Abwandlung, bei der die Vorrichtung zumindest zwei relativ zueinander bewegliche Kompartimente hat. Dabei ist die Bestimmung der Relativposition nicht auf benachbarte Kompartimente beschränkt, sondern es kann zudem auch die Relativposition in Wirkverbindung tretender, einander gegenüberliegender Kompartimente bestimmt werden.

Dabei kann die Vorrichtung bei beliebigen Gelenken eingesetzt werden. Besonders vorteilhaft ist es hingegen, wenn die Vorrichtung zur Einstellung entweder einer der Tibia innen zugeordneten Gelenkpfanne dient oder dem Femurgelenkkopf außen tibiaseits in der Hauptfunktionsebene sagittal eine Konvexität und transversal eine Konkavität zugeordnet ist.

Die zweitgenannte Aufgabe, wird gelöst durch ein Verfahren zu Bestimmung einer relativen Sollposition eines insbesondere als Endoprothese für das menschliche Kniegelenk ausgeführten künstlichen Gelenkelementes, bei dem zunächst zumindest ein Kompartiment entfernt und eine als Einstellhilfe dienende Vorrichtung lösbar fixiert wird, anschließend das Kniegelenk bewegt wird und die sich aus der relativen Verlagerung der Einstellhilfe ergebenden Referenzposition erfasst und diese der Auswahl und/oder Einstellung des einzusetzenden künstlichen Gelenkelementes zugrunde gelegt wird und schließlich die Vorrichtung entfernt wird, um das künstliche Kniegelenk einzusetzen.

Hierdurch wird eine messtechnische Erfassung der Gelenkparameter des menschlichen Gelenkes zur Anpassung und Auswahl des geeigneten künstlichen Gelenkes entbehrlich, weil die Referenzposition in einfacher Weise an der Vorrichtung abgelesen werden kann und unmittelbar zur Anpassung und Auswahl der künstlichen Gelenkelemente herangezogen werden kann.

Lediglich eine einfache von Hilfspersonen eingeleitete Bewegung des Gelenkes genügt, durch die sich die Referenzposition selbsttätig einstellt. Besonders zweckmäßig ist es dabei, wenn das Kniegelenk zur Erzielung der Referenzposition insbesondere während der Operation vollständig gebeugt wird.

Dabei können gemäß einer weiteren, besonders vorteilhaften Abwandlung, bei der das Verfahren zur Bestimmung der relativen Sollposition mehrerer Kompartimente des Gelenkkopfs oder der Gelenkpfanne wiederholt wird, die Relativpositionen mehrerer oder aller Gelenkkompartimente problemlos eingestellt werden.

Beispielswiese werden zur Bestimmung des geeigneten Kompartimentes die Referenzposition als eine Korrekturgröße erfasst und die Gelenkkompartimente des Ersatzgelenkes auf der Basis der Referenzposition bestimmt.

Die Erfindung lässt verschiedene Ausführungsformen zu. Zur weiteren Verdeutlichung ihres Grundprinzips sind zwei davon in den Zeichnungen dargestellt und werden nachfolgend beschrieben. Diese zeigen in
- Fig.1: eine laterale Ansicht einer erfindungsgemäßen Einstellvorrichtung mit einem Freiheitsgrad; die gestrichelte Seite stellt die wahlweise laterale oder mediale Anordnung des bewegliche Kompartimentes dar;
- Fig.2: eine frontale Ansicht der in Figur 1 gezeigten Einstellvorrichtung;
- Fig.3: eine laterale Ansicht einer weiteren Einstellvorrichtung mit einem Freiheitsgrad;
- Fig.4: eine frontale Ansicht der in Figur 3 gezeigten Einstellvorrichtung.

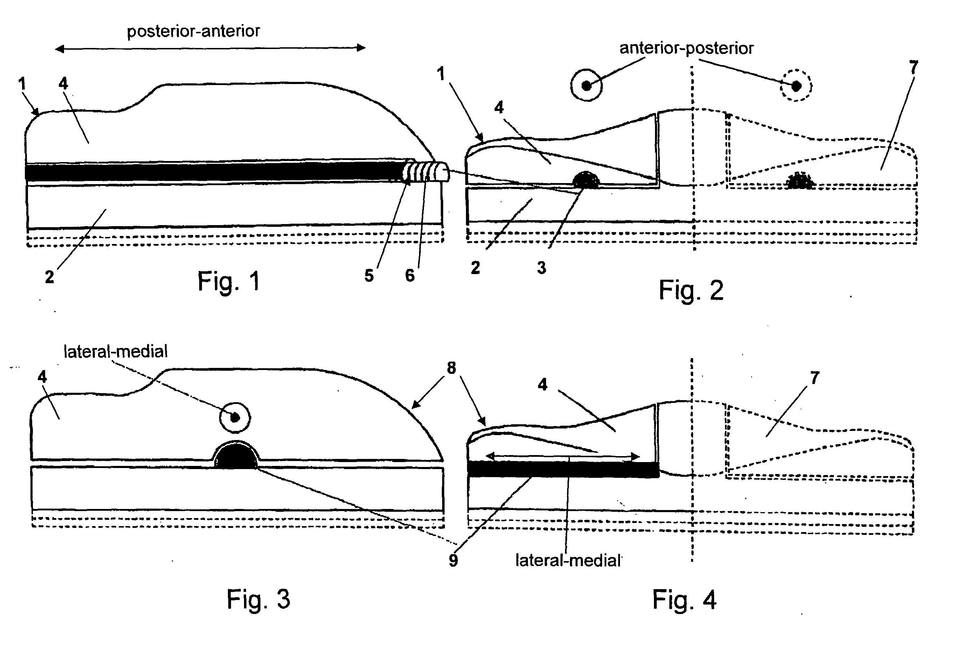

Die Figuren 1 und 2 zeigen eine laterale sowie eine frontale Ansicht einer in ihrer Gesamtheit aller dargestellten Elemente mit 1 bezeichneten Vorrichtung zur Bestimmung und Einstellung der optimalen Position einer nicht gezeigten Funktionsfläche eines künstlichen Gelenkes und der dementsprechend gestalteten Implantat-Komponenten des künstlichen Gelenkes. Die Vorrichtung 1 ist mit einem relativ zu einer an einem menschlichen Gelenk oder Knochenbereich fixierbaren Aufnahme 2 mittels einer Schiebeführung 3 beweglichen Kompartiment 4 ausgestattet. Eine sich während der Operation durch die posterior - anterior Bewegung des Kniegelenkes einstellende Referenzposition der Aufnahme 2 gegenüber dem Kompartiment 4 ist dabei mittels einer eine Skalierung 5 aufweisenden Markierung 6 erfassbar. Dadurch wird die Möglichkeit geschaffen, die optimale Position zwecks Übertragung auf die Abmessungen der Implantat-Komponenten und Implantat-Position abzulesen. Zusätzlich ist das laterale Kompartiment 4 auch relativ zu einem medialen Kompartiment 7 beweglich, um so die Einstellbarkeit zu verbessern.

Demgegenüber zeigen die Figuren 3 und 4 eine laterale sowie eine frontale Ansicht einer weiteren in ihrer Gesamtheit aller dargestellten Elemente mit 8 bezeichneten Vorrichtung, wobei das laterale Kompartiment 4 und/oder das mediale Kompartiment 7 in Richtung von zwei Raumachsen posterior N anterior sowie lateral N medial nach Art eines Kreuztisches mittels jeweils der in den Figuren 1 und 2 gezeigten Schiebeführung 3 und einer weiteren Schiebeführung 9 beweglich angeordnet ist. Die Vorrichtung 8 wird als Einstellhilfe lösbar fixiert, wobei das laterale und/oder das mediale Kompartiment 4, 7 relativ zu der Aufnahme 2 beweglich sind. Somit führt die Einleitung der Gelenkbewegung zu einer Verlagerung in die optimale Relativposition, die als Referenzposition an der Vorrichtung 8 erkennbar oder arretierbar ist, so dass das künstliche Gelenk aufgrund dieser Referenzposition ausgewählt bzw. angepasst werden kann.

## Patentansprüche

1. Eine insbesondere zur Anwendung bei einer Endoprothese für das menschliche Kniegelenk bestimmte Vorrichtung (1, 8) zur Bestimmung und/oder Einstellung der optimalen Relativposition mehrerer Funktionsflächen eines Gelenkkopfs und/oder einer Gelenkpfanne des menschlichen und/oder eines künstlichen Gelenkes, insbesondere dementsprechend gestalteten Implantat-Komponenten, wobei die Vorrichtung (1, 8) mit einem relativ zu einer an einem menschlichen Gelenk oder Knochenbereich lösbar fixierbaren Aufnahme (2) beweglichen Kompartiment (4, 7) ausgestattet ist, so dass eine sich durch die Bewegung des Kniegelenkes einstellende Referenzposition des Kompartiments (4, 7) gegenüber der Aufnahme (2) erfassbar ist.

2. Vorrichtung (8) nach Anspruch 1, **dadurch gekennzeichnet, dass** das mediale Gelenkkompartiment aus Gelenkpfanne und Gelenkkopf besteht und das laterale Gelenkkompartiment in sagittaler Richtung konvex-konvex und der Gelenkkopf in transversaler Richtung konvex und die Gelenkpfanne konkav gestaltet sind.

3. Vorrichtung (1, 8) nach den Ansprüchen 1 oder 2, **dadurch gekennzeichnet, dass** das Kompartiment (4, 7) eine Kontaktlinie im Transversalschnitt jeweils auf inneren Abhängen des Gelenkkopfs und/oder der Gelenkpfanne liegt.

4. Vorrichtung (1, 8) nach zumindest einem der vorhergehenden Ansprüche, **gekennzeichnet durch** eine Einstellbarkeit vertikal zu einer relativen Beweglichkeit der Kompartiment (4, 7).

5. Vorrichtung (1, 8) nach zumindest einem der vorhergehenden Ansprüche, **dadurch gekennzeichnet, dass** das Kompartiment (4, 7) in Richtung von zwei Raumachsen (posterior N anterior; lateral N medial) beweglich angeordnet ist.

6. Vorrichtung (1, 8) nach zumindest einem der vorhergehenden Ansprüche, **dadurch gekennzeichnet, dass** das Kompartiment (4, 7) mittels einer Schiebeführung (3, 9) mit der Aufnahme (2) verbunden ist.

7. Vorrichtung (1, 8) nach zumindest einem der vorhergehenden Ansprüche, **dadurch gekennzeichnet, dass** das Kompartiment (4, 7) eine insbesondere nutenförmige Ausnehmung aufweist, in welche ein Vorsprung der Aufnahme (2) eingreift.

8. Vorrichtung (1, 8) nach zumindest einem der vorhergehenden Ansprüche, **dadurch gekennzeichnet, dass** die Referenzposition durch eine Markierung (6) identifizierbar ist.

9. Vorrichtung (1, 8) nach Anspruch 8, **dadurch gekennzeichnet, dass** die Markierung (6) eine Skalierung (5) aufweist.

10. Vorrichtung (1, 8) nach Anspruch 8 oder 9, **dadurch gekennzeichnet, dass** der Markierung (6) ein Sensor zur elektronischen Signalverarbeitung zugeordnet ist.

11. Vorrichtung (1, 8) nach zumindest einem der vorhergehenden Ansprüche, **dadurch gekennzeichnet, dass** die Vorrichtung (1, 8) einen Sensor zur Erfassung der Relativbewegung des Kompartimentes (4, 7) gegenüber der Aufnahme (2) während der Bewegung des Kniegelenks aufweist.

12. Vorrichtung (1, 8) nach zumindest einem der vorhergehenden Ansprüche, **dadurch gekennzeichnet, dass** die sich durch die Bewegung des Kniegelenkes einstellende Referenzposition anschließend arretierbar ist.

13. Vorrichtung (1, 8) nach zumindest einem der vorhergehenden Ansprüche, **dadurch gekennzeichnet, dass** die Vorrichtung (1, 8) zumindest zwei relativ zueinander bewegliche Kompartimente (4, 7) hat.

14. Vorrichtung (1,8) nach zumindest einem der vorhergehenden Ansprüche, **dadurch gekennzeichnet, dass** die Vorrichtung (1, 8) zur Einstellung einer der Tibia zugeordneten Gelenkpfanne dient.

15. Vorrichtung (1,8) nach zumindest einem der vorhergehenden Ansprüche, **dadurch gekennzeichnet, dass** die Vorrichtung (1, 8) zur Einstellung eines der Tibia zugeordneten Gelenkkopfs (Sagittalschnitt) dient.

16. Verfahren zu Bestimmung einer relativen Sollposition eines insbesondere als Endoprothese für das menschliche Kniegelenk ausgeführten künstlichen Gelenkelementes, bei dem zunächst zumindest ein Kompartiment entfernt und eine als Einstellhilfe dienende Vorrichtung lösbar fixiert wird, anschließend das Kniegelenk bewegt wird und die sich aus der relativen Verlagerung der Einstellhilfe ergebenden Referenzposition erfasst und diese der Auswahl und/oder Einstellung des einzusetzenden künstlichen Gelenkelementes zugrunde gelegt wird und schließlich die Vorrichtung entfernt wird, um das künstliche Kniegelenk einzusetzen.

17. Verfahren nach Anspruch 16, **dadurch gekennzeichnet, dass** das Kniegelenk zur Erzielung der Referenzposition vollständig gebeugt wird.

18. Verfahren nach Anspruch 16 oder 17, **dadurch gekennzeichnet, dass** das Verfahren zur Bestimmung der relativen Sollposition mehrere Kompartiment des Gelenkkopfs und/oder der Gelenkpfanne wiederholt wird.

19. Verfahren nach zumindest einem der Ansprüche 16 bis 18, **dadurch gekennzeichnet, dass** die Referenzposition als eine Korrekturgröße erfasst wird.

20. Verfahren nach zumindest einem der Ansprüche 16 bis 19, **dadurch gekennzeichnet, dass** die Gelenkkompartiment des Ersatzgelenkes auf der Basis der Referenzposition bestimmt werden.

21. Implantat-Teile, die auf der Basis der Funktionsweise der Vorrichtung nach zumindest einem der Ansprüche 1 bis 15 gestaltet sind und mit optimaler Gestalt implantiert werden.
